# EUROPÄISCHE PATENTSCHRIFT

(11) **EP 3 171 676 B1**
(45) Veröffentlichungstag und Bekanntmachung des Hinweises auf die Patenterteilung: **24.06.2020**
(21) Anmeldenummer: 15195015.1
(22) Anmeldetag: 17.11.2015
(51) Int. Cl.: H05H 1/24, A61L 2/14, A61B 18/04

(54) **PLASMAERZEUGUNGSVORRICHTUNG, PLASMAERZEUGUNGSSYSTEM UND VERFAHREN ZUR ERZEUGUNG VON PLASMA**
PLASMA GENERATING DEVICE, PLASMA GENERATING SYSTEM AND METHOD OF GENERATING PLASMA
DISPOSITIF DE PRODUCTION DE PLASMA, SYSTEME DE PRODUCTION DE PLASMA ET PROCEDE DE PRODUCTION DE PLASMA

(43) Veröffentlichungstag der Anmeldung: 24.05.2017
(73) Patentinhaber: Leibniz-Institut für Plasmaforschung und Technologie e.V., 17489 Greifswald (DE)
(72) Erfinder: Prof. Dr. Weltmann, Klaus-Dieter, 18609 Binz (DE); Dr. Brandenburg, Ronny, 17495 Groß Kiesow (DE); Dr. Stieber, Manfred, 17489 Greifswald (DE); Horn, Stefan, 17438 Wolgast (DE); Turski, Phillipp, 17489 Greifswald (DE)
(74) Vertreter: Schulz Junghans Patentanwälte PartGmbB

(56) Entgegenhaltungen:
- WO-A1-2010/138102
- DE-A1-102013 203 648
- DE-U1-202009 011 521
- US-A1- 2006 133 970
- US-A1- 2014 182 879

## Beschreibung

Die vorliegende Anmeldung betrifft eine Plasmaerzeugungsvorrichtung mit einer Hochspannungselektrode sowie mindestens einer Außenelektrode. Des Weiteren betrifft die vorliegende Erfindung ein Plasmaerzeugungssystem mit mehreren erfindungsgemäßen Plasmaerzeugungsvorrichtungen, sowie ein Verfahren zur Erzeugung von Plasma mittels einer erfindungsgemäßen Plasmaerzeugungsvorrichtung oder einem erfindungsgemäßen Plasmaerzeugungssystem.

Die Plasmaerzeugungsvorrichtung dient insbesondere zur flächigen, antimikrobiellen Behandlung feuchter Oberflächen und bietet damit eine Möglichkeit der antimikrobiellen Wundbehandlung in feuchtem Milieu mittels spezieller Atmosphärendruckplasmaquellen.

Atmosphärendruckplasmaquellen können für medizinische, zahnmedizinische oder kosmetische Zwecke eingesetzt werden. Übliche Plasmaerzeugungseinrichtungen sehen eine Superposition von Jet-Plasmen für die Abdeckung einer Fläche vor. Es werden dabei verschiedene Jet-Düsengeometrien genutzt.

Neben der punktuellen Anwendung wird versucht, Plasmen auch flächig zu nutzen, wie zum Beispiel zur Behandlung von Wunden. Mit derartigen flächig ausgeführten Einrichtungen wird üblicherweise eine dielektrisch behinderte Entladung ("dielectric barrier discharge" - DBD) realisiert. Dabei unterscheidet man zwischen zwei unterschiedlichen Elektrodenkonfigurationen: einer Anordnung zur Erzeugung einer dielektrisch behinderten Oberflächenentladung (SBD), bei der die Elektroden beiderseits eines Dielektrikums direkt auf der Oberfläche des Dielektrikums angeordnet sind und das Plasma auf der Oberfläche des Dielektrikums ausgebildet wird, und einer Anordnung zur Erzeugung einer dielektrisch behinderten Volumenentladung (VBD), bei der die beiden Elektroden (mindestens eine mit einem Dielektrikum) in einem geringen Abstand voneinander angeordnet sind und sich das Plasma in diesem Raum zwischen den beiden Elektroden ausbildet.

Derartige Einrichtungen sind meistens aus Festigkeitsgründen aus einem relativ festen bzw. starren Grundmaterial ausgeführt. Eine Anpassung an bestimmte Topografien ist daher nur eingeschränkt möglich.

Insbesondere Plasmaerzeugungseinrichtungen, die zur Anwendung im medizinischen Bereich ausgestaltet sind, unterliegen relativ strengen Vorschriften hinsichtlich der Sicherheit gegenüber einer drohenden, aus der anliegenden Hochspannung resultierenden Verletzungsgefahr sowie hinsichtlich der Einhaltung der Richtlinien der elektromagnetischen Verträglichkeit (EMV).

Eine flächige Plasmaerzeugungsvorrichtung ist beispielsweise aus der WO 2011/023478 A1 bekannt. Bei dieser vorbekannten Vorrichtung handelt es sich um eine Manschette zur Behandlung menschlicher oder tierischer Haut mit Hilfe eines kalten Atmosphärendruckplasmas. Die Manschette besteht aus einer Hochspannungselektrode, einem isolierenden Elastomer, einem Dielektrikum und einer geerdeten Elektrode. Die flächige Hochspannungselektrode wird zum einen durch ein flächiges, isolierendes Elastomer und zum anderen durch ein flächiges Dielektrikum begrenzt. Auf der gegenüberliegenden Seite des Dielektrikums befindet sich eine flächige, geerdete Elektrode. Bei Anlegen einer Hochspannung führt diese Anordnung zu einer dielektrisch behinderten Oberflächenentladung. Für die Nutzung der Manschette auf erkrankter Haut des menschlichen Körpers sind die Hochspannungselektrode, das isolierende Elastomer, das Dielektrikum und die Elektrode flexibel und lassen sich auf beliebig gekrümmte Oberflächen auflegen.

Eine weitere Plasmaerzeugungsvorrichtung wird in der WO 2013/167693 A1 offenbart. Auch diese offenbart eine Vorrichtung zur Behandlung von Bereichen menschlicher oder tierischer Oberflächen mittels eines kalten Atmosphärendruckplasmas durch Erzeugung einer dielektrisch behinderten Oberflächenentladung. Diese Plasmaerzeugungsvorrichtung der WO 2013/167693 A1 besteht aus einer drahtförmigen Hochspannungselektrode mit anliegender geerdeter Elektrode. Für die dielektrisch behinderte Oberflächenentladung ist die Hochspannungselektrode mit einem Dielektrikum ummantelt, welches eine flexible Einheit bildet. Die geerdete Elektrode besteht aus einem leitenden, textilen Material. Damit passt sich die Plasmaerzeugungsvorrichtung der Kontur zu behandelnder Körperareale an.

Die Verwendung der oben genannten Plasmaerzeugungsvorrichtungen ist für die Wundheilung biologischer Gewebe in feuchtem Milieu nachteilig. Die Vorrichtungen der WO 2011/023478 A1 sowie die WO 2013/167693 A1 basieren auf der Erzeugung einer dielektrisch behinderten Oberflächenentladung. Diese Art der Erzeugung wird wesentlich beeinträchtigt oder sogar völlig unterdrückt durch hohe Feuchtigkeit auf der Oberfläche des Dielektrikums.

Der Beeinträchtigung durch hohe Feuchtigkeit lässt sich durch die Verwendung einer dielektrisch behinderten Volumenentladung entgegenwirken. Eine auf einer dielektrisch behinderten Volumenentladung basierende Plasmaerzeugungsvorrichtung ist in der WO 2011/076193 A1 offenbart. Die Vorrichtung der WO 2011/076193 A1 besteht aus einer flächigen Hochspannungselektrode, die auf der Vorder- und auf der Rückseite jeweils durch ein flächiges Dielektrikum begrenzt wird. Diese Elektrodenanordnung ist flexibel an die Kontur der Oberfläche anpassbar. Die Oberfläche des vorderseitigen Dielektrikums ist durch Noppen mit äquidistanter Höhe geprägt. In Verbindung mit einer geerdeten, elektrisch leitenden Oberfläche wird in den Luftführungsbereichen zwischen den Noppen eine dielektrisch behinderte Volumenentladung erzeugt.

Die Nutzung der zu behandelnden Oberfläche als geerdete Gegenelektrode birgt jedoch den Nachteil von unkontrollierbaren bzw. undefinierbaren Oberflächeneffekten auf dem zu behandelnden biologischen Gewebe. Ferner liegen die als Abstandshalter dienenden Noppen direkt auf der zu behandelnden Wunde und verursachen unerwünschte Reizungen oder rasterförmige Dekontaminationen.

WO 2010/138102 A1 offenbart eine Plasmaerzeugungsvorrichtung nach dem Oberbegriff des Anspruchs 1.

Die aus dem Stand der Technik bekannten Plasmaerzeugungsvorrichtungen mit flächigen Atmosphärendruck-Plasmaquellen weisen für die medizinische Nutzung Nachteile auf. Eine Nutzung von Atmosphärendruck-Plasmaquellen für medizinische, zahnmedizinische oder kosmetische Zwecke ist nur dann möglich, wenn die Einhaltung der Richtlinien der elektromagnetischen Verträglichkeit (EMV) gewährleistet werden kann. Bei den oben genannten flächigen Plasmaquellen ist die Störstrahlung durch die Plasmaquelle selbst nicht mehr zu vernachlässigen, die erforderlichen EMV-Anforderungen werden nicht erfüllt.

Der vorliegenden Erfindung liegt die Aufgabe zu Grunde, eine Plasmaerzeugungsvorrichtung, ein Plasmaerzeugungssystem sowie ein Verfahren zur Erzeugung von Plasma zur Verfügung zu stellen, mit denen in einfacher, kostengünstiger, sicherheitstechnisch unbedenklicher sowie flexibel anwendbarer Weise eine Behandlung von Oberflächen, insbesondere eine Desinfektion bzw. Sterilisation von Oberflächen wie z. B. auch von biologischem Gewebe, möglich ist.

Diese Aufgabe wird durch die erfindungsgemäße Plasmaerzeugungsvorrichtung nach Anspruch 1 sowie durch das erfindungsgemäße Plasmaerzeugungssystem nach Anspruch 12 und durch das erfindungsgemäße Verfahren zur Erzeugung von Plasma nach Anspruch 13 gelöst. Vorteilhafte Ausgestaltungsformen der erfindungsgemäßen Plasmaerzeugungsvorrichtung sind in den Unteransprüchen 2 bis 11 angegeben.

Die erfindungsgemäße Plasmaerzeugungsvorrichtung dient insbesondere zur Erzeugung eines Atmosphärendruckplasmas zur antimikrobiellen Behandlung feuchter Oberflächen. Die Plasmaerzeugungsvorrichtung umfasst eine Hochspannungselektrode sowie mindestens eine Außenelektrode, wobei die Hochspannungselektrode zumindest in einer Koordinatenrichtung zwischen Leitungsmaterial wenigstens einer Außenelektrode angeordnet ist. Die Hochspannungselektrode ist zumindest auf einer einer Außenelektrode zugewandten Seite mit einem Dielektrikum bedeckt. Gegebenenfalls ist die Hochspannungselektrode vollständig mit einem Dielektrikum bedeckt. Zwischen der jeweiligen Außenelektrode und der Hochspannungselektrode ist über deren Längserstreckung wenigstens ein Abstandselement angeordnet, welches zumindest im Bereich dessen Anordnung die jeweilige Außenelektrode von der Hochspannungselektrode elektrisch isoliert und in einem konstanten Abstand positioniert.

Das der Hochspannungselektrode in zumindest einer Koordinatenrichtung gegenüberliegende Leitungsmaterial der Außenelektrode dient bei Anlage einer geeigneten elektrischen Spannung an die Plasmaerzeugungsvorrichtung als die eigentliche Außenelektrode.

Vorzugsweise ist das Dielektrikum nicht nur an der der jeweiligen Außenelektrode zugewandten Seite der Hochspannungselektrode vorhanden, sondern umschließt die damit versehene Hochspannungselektrode.

Durch das Abstandselement bzw. durch die Anordnung mehrerer Abstandselemente wird eine im Wesentlichen äquidistante Anordnung der Elektroden zueinander realisiert. Daraus ergibt sich die Möglichkeit der Erzeugung einer dielektrisch behinderten Volumenentladung, die wesentlich stabiler gegenüber einem stark feuchten Milieu ist, sodass die erfindungsgemäße Plasmaerzeugungsvorrichtung insbesondere vorteilhaft an Wunden bzw. biologischen Geweben einsetzbar ist. Darüber hinaus wird durch die beidseitig der Hochspannungselektrode angeordnete Außenelektrode bzw. Außenelektroden eine wesentliche Reduzierung der von der Plasmaquelle abgestrahlten elektromagnetischen Störstrahlung erreicht.

Die Hochspannungselektrode muss für den Hochvolt-(HV)-Bereich ausgebildet sein , nämlich für eine Spannung von mindestens 1 kV. In einer Ausgestaltung der erfindungsgemäßen Plasmaerzeugungsvorrichtung ist das Dielektrikum der Hochspannungselektrode für die doppelte Spannungsfestigkeit (also mindestens 2 kV) ausgelegt. Dabei kann die Plasmazeugungsvorrichtung für einen Spannungsbereich von 1 kV bis zu 25 kV eingerichtet sein, bei einer Frequenz der Wechselspannung von 1 kHz Kilohertz bis zu 1 Mhz, wobei die Ansteuerung sowohl mit Sinus-, Rechteck- oder Dreiecksignal als auch gepulst erfolgen kann.

Die Außenelektrode bzw. Außenelektroden sind geerdet ausgeführt. In einer Ausgestaltung der erfindungsgemäßen Plasmaerzeugungsvorrichtung umfasst diese eine erste Außenelektrode und eine zweite Außenelektrode, wobei die auch als Innenelektrode bezeichnete Hochspannungselektrode zwischen den beiden Außenelektroden angeordnet ist.

In diesem Fall ist die Hochspannungselektrode auf den den beiden Außenelektroden zugewandten Seiten mit einem Dielektrikum bedeckt.

Die Hochspannungselektrode kann als ein Leitungselement ausgeführt sein, dessen Verhältnis der Länge zum Durchmesser wenigstens 10 beträgt. Ein solches dünnes Leitungselement kann ein isolierter dünner Draht oder auch ein isolierter, elektrisch leitender Faden sein.

Die Hochspannungselektrode kann als ein isolierter Draht ausgeführt sein, wobei die Isolation dabei gleichzeitig das Dielektrikum ausbildet.

In einer Ausgestaltung der Plasmaerzeugungsvorrichtung weist die Hochspannungselektrode einen mäanderförmigen oder spiralförmigen Verlauf auf.

In einer weiteren Ausführungsalternative kann die Hochspannungselektrode und/oder die Außenelektrode bzw. Außenelektroden als elektrisch leitfähiges textiles Material in Form eines Geflechts, Gewebes, Gewirkes oder Gestricktes oder auch ein Nähgewirk, Vliesstoff oder Filz oder eine Kombination dieser textilen Materialien sein, wobei das verwendete Material Metall oder auch lediglich eine Metallbeschichtung ist und im Fall der Hochspannungselektrode dieses Material mit einem Dielektrikum beschichtet ist.

In einer weiteren Ausführungsform ist die Außenelektrode als elektrisch leitfähige Folie ausgebildet. Dabei ist die Folie insbesondere perforiert und/oder strukturiert ausgebildet.

Hinzukommend oder alternativ kann auch die Hochspannungselektrode als eine derartige, mit einem Dielektrikum beschichtete, Folie ausgestaltet sein.

Das jeweilige, die Hochspannungselektrode sowie auch die Außenelektrode oder Außenelektroden sowie ggf. auch das Dielektrikum, eine Isolation und die Abstandselemente ausbildende, Material kann einen Elastizitätsmodul von maximal 1 x10⁹ N/m², insbesondere einen Elastizitätsmodul zwischen 0,1 x10⁹ N/m² und 0,5 x10⁹ N/m² aufweisen, sodass auch die gesamte Vorrichtung diesen Elastizitätsmodul und somit eine sehr geringe Biege- und/ oder Torsionssteifigkeit aufweist. Die Verwendung von elastischen oder flexiblen Materialien ermöglicht die flexible Anpassung der Plasmazeugungsvorrichtung an unterschiedlichste Topografien.

Die Außenelektrode ist in einer Ausgestaltung der Plasmaerzeugungsvorrichtung als eine die Hochspannungselektrode einschließende, gasdurchlässige und flexible Elektrode ausgestaltet, wie z. B. als ein Metallgewebe oder auch ein anderes elektrisch leitfähiges, textiles Material oder eine perforierte, elektrisch leitende Elastomer-Folie bzw. eine mit einem elektrisch leitfähigen Material beschichtete Elastomer-Folie, wie z. B. eine Silikonfolie.

Die Außenelektrode ist geerdet ausgeführt und kann auch ein Schlauch aus einem perforierten oder gewebten leitfähigen Material oder aus spiralförmig angeordnetem dünnen Edelstahl sein. Die in der erfindungsgemäßen Plasmaerzeugungsvorrichtung genutzten Außenelektroden sind leitfähig miteinander verbunden.

In einer weiteren Ausführungsform der erfindungsgemäßen Plasmaerzeugungsvorrichtung ist vorgesehen, dass die Plasmaerzeugungsvorrichtung mehrere Abstandselemente aufweist, die als die Hochspannungselektrode zumindest abschnittsweise umschließende Isolierstücke ausgeführt sind. Derartige, auch Distanzstücke bezeichnete Abstandselemente können z. B. Silikonschlauchstücke sein. In einer Ausführungsform ist vorgesehen, dass ein Abstandselement oder auch mehrere Abstandselemente die Hochspannungselektrode über deren gesamten Verlauf umschließen.

In der Plasmaerzeugungsvorrichtung der vorliegenden Erfindung ist vorgesehen, dass das Abstandselement flächig ausgebildet ist. Das bedeutet, dass sich das Abstandselement im Wesentlichen in einer geraden Ebene erstreckt, sodass es die sich vorzugsweise ebenfalls in geraden, flächigen Ebenen erstreckenden Außenelektroden sowie die dazwischen aufgenommene Hochspannungselektrode voneinander separiert.

Das Abstandselement ist in der vorliegenden Erfindung durch eine perforierte und/oder strukturierte gasdurchlässige vorzugsweise flächendeckende Folie aus einem elektrisch nicht leitfähigen Elastomer realisiert. Solche Folie, wie z. B. eine Silikonfolie, kann auch als Dielektrikum verwendet werden, wobei eine drahtförmige Hochspannungselektrode, die z.B. auch als Geflecht, Gewebe, Gewirk oder Gestrick vorliegt, in diese Folie eingebettet ist.

Eine solche Folie kann Öffnungen aufweisen, sodass die als Dielektrikum dienende Folie eine Gasdurchlässigkeit aufweist. Die Öffnungen können durch eine Perforation hergestellt sein.

Diese Ausgestaltung sollte die Folie insbesondere dann aufweisen, wenn die Folie flächendeckend zwischen Elektroden ausgeführt ist.

In einer weiteren Ausführungsvariante der Plasmaerzeugungsvorrichtung ist vorgesehen, dass das Abstandselement als ein Geflecht, Gewebe, Gewirk oder Gestrick ausgebildet ist. Dadurch sind Öffnungen im Abstandselement integriert. Alternativ kann vorgesehen sein, dass auf einem solchen Abstandselement punktförmige oder rasterförmige Beschichtungen erzeugt sind, um den für eine dielektrisch behinderte Volumenentladung benötigten Abstand zwischen den Elektroden einhalten zu können.

In ergänzender Ausführungsform umfasst die erfindungsgemäße Plasmaerzeugungsvorrichtung eine Spannungsversorgungseinheit, die dazu eingerichtet ist, zwischen der Außenelektrode bzw. den Außenelektroden und der Hochspannungselektrode eine nieder- bis hochfrequente Hochspannung anzulegen.

Vorteilhafte Ausgestaltungen der Plasmaerzeugungsvorrichtung hinsichtlich des Materials der Elektroden sowie auch hinsichtlich der Spannungsversorgungseinheit, der Zuleitungen und Anschlüsse der Leitungen an den Elektroden sind in den Dokumenten WO2011023478A1 sowie WO2013167693A1 gelehrt.

Eine generelle Ausgestaltungsform der erfindungsgemäßen Plasmaerzeugungsvorrichtung besteht somit darin, dass die Plasmaerzeugungsvorrichtung im Wesentlichen flächig ausgeführt ist, wobei eine flächige Hochspannungselektrode sandwich-artig von zwei flächig ausgeführten Außenelektroden umgeben ist.

Eine weitere Ausführungsform besteht in einer stab- bzw. rohrförmigen Ausgestaltung, wobei eine länglich erstreckte Hochspannungselektrode von einer wendel- bzw. schraubengangartig geformten Außenelektrode umgeben ist. Eine weitere Alternative besteht darin, dass eine längliche Hochspannungselektrode von einer rohrförmigen Außenelektrode aus einem gasdurchlässigen, elektrisch leitfähigen Material umgeben ist. Wird bei diesen Ausführungsformen flexibles Material verwendet, können diese spiral- oder mäanderförmig flächig angeordnet werden, so dass auch in diesen Fällen eine Anpassung an bestimmte Topografien möglich ist.

Allen Ausgestaltungsform ist gemeinsam, dass zwischen Hochspannungselektrode und Material der Außenelektrode Abstandselemente angeordnet sind, sodass jeweils ein Abstand besteht zwischen Hochspannungselektrode und Außenelektrode zur Realisierung einer dielektrisch behinderten Volumenentladung.

Dadurch wird eine effektive, antimikrobielle Plasmabehandlung von Wunden und biologischen Geweben ermöglicht, sogar in einem sehr feuchten Milieu. Durch die beidseitig der Hochspannungselektrode angeordnete Außenelektrode oder Außenelektroden wird eine wesentliche Reduzierung der von der Plasmaquelle abgestrahlten elektromagnetischen Störstrahlung erreicht. Dies ermöglicht, auch für flächige Plasmaquellen die Bedingungen zur Einhaltung der Richtlinien der elektromagnetischen Verträglichkeit (EMV) zu erfüllen, sodass eine erfindungsgemäße Plasmavorrichtung auch für den Einsatz zu medizinischen, zahnmedizinischen und kosmetischen Zwecken zugelassen werden kann.

Im Gegensatz zu Plasmaquellen, die auf einer dielektrisch behinderten Oberflächenentladung basieren, bei denen die Ausbildung eines Plasmas auf der Oberfläche des Dielektrikums durch hohe Feuchtigkeit wesentlich beeinträchtigt oder sogar unterdrückt wird, ist die vorliegende Plasmaerzeugungsvorrichtung aufgrund der dielektrisch behinderten Volumenentladung wesentlich feuchtigkeitstauglicher und somit auch für die effektive, antimikrobielle Plasmabehandlung von Wunden und biologischen Geweben in sehr feuchtem Milieu geeignet.

Im Gegensatz zu Plasmaquellen, die zwar die dielektrisch behinderte Volumenentladung ausführen, jedoch die Oberfläche des zu behandelnden biologischen Gewebes als geerdete Gegen- bzw. Außenelektrode nutzen und damit das Gewebe durch unkontrollierte bzw. undefinierte Oberflächeneffekte gefährden (wie z. B. im Fall eines elektrischen Durchschlags der Isolierung der Hochspannungselektrode), gewährleistet die erfindungsgemäße Plasmaerzeugungsvorrichtung durch die beidseitig der Hochspannungselektrode vorgesehene Anordnung wenigstens einer Außenelektrode die Eliminierung dieser Gefahr.

Zur optimalen Wundverträglichkeit kann die erfindungsgemäße Plasmaerzeugungsvorrichtung eine gasdurchlässige Wundauflage umfassen, die bei der Wundbehandlung zwischen biologischem Gewebe und der Plasmaerzeugungsvorrichtung zu positionieren ist. Eine derartige Wundauflage kann eine dünne, relativ weitmaschige textile Wundauflage (z.B. steriler Verbandsmull)sein. Derart wird verhindert, dass die Plasmaquelle direkt mit der Wunde in Kontakt kommt und es zu unerwünschten Reizungen oder auch zu einer Kontamination der Wunde wie bei der direkten Auflage nicht steriler Plasmaerzeugungsvorrichtungen kommt.

Ein weiterer Aspekt der vorliegenden Erfindung ist ein Plasmaerzeugungssystem, welches insbesondere zur großflächigen Behandlung von Oberflächen, wie z. B. von Brandwunden, geeignet ist. Dieses Plasmaerzeugungssystem umfasst mehrere erfindungsgemäße Plasmaerzeugungsvorrichtungen sowie eine oder mehrere Spannungsversorgungseinheiten, die mit den Plasmaerzeugungsvorrichtungen elektrisch verbindbar oder verbunden sind.

Insbesondere lassen sich die Plasmaerzeugungsvorrichtungen des Plasmaerzeugungssystems matrix-artig anordnen oder auch linear anordnen, um entsprechend geformte bzw. dimensionierte Oberflächenbereiche abdecken zu können. Es lassen sich somit mehrere erfindungsgemäße Plasmaerzeugungsvorrichtungen zu einer größeren Plasmaquelle zusammenfügen, um z. B. größere Hautbereiche von Brandopfern schnell, flexibel sowie medizinisch unbedenklich behandeln zu können. Zur Spannungsversorgung gibt es dabei die Möglichkeit, eine einzelne leistungsfähige Spannungsquelle als auch mehrere identische Spannungsquellen zur Versorgung eines einzelnen Flächenelementes und damit auch zur Versorgung einer jeweiligen Spannungserzeugungsvorrichtung zu verwenden.

Die vorliegende Erfindung wird ergänzt durch ein Verfahren zur Erzeugung von Plasma, insbesondere zur Erzeugung einer dielektrisch behinderten Volumenentladung, bei der eine erfindungsgemäße Plasmaerzeugungsvorrichtung oder auch ein erfindungsgemäßes Plasmaerzeugungssystem zur Verfügung gestellt wird und eine elektrische Hochspannung an die Hochspannungselektrode und mindestens eine Außenelektrode angelegt wird. Dadurch lässt sich insbesondere eine Desinfektion von Oberflächen, wie z. B. Wunden menschlichen oder tierischen Gewebes, durchführen, bei dem das Verfahren zur Erzeugung von Plasma in einem solchen Abstand zur zu desinfizierenden Oberfläche durchgeführt wird, dass die zu desinfizierende Oberfläche mit den durch das Plasma erzeugten reaktiven Spezies des Plasmagases in Kontakt kommt.

Weitere Einzelheiten und Vorteile der vorliegenden Erfindung ergeben sich aus der nachfolgenden Beschreibung von Ausführungsbeispielen in Verbindung mit der Zeichnung.

Die Figuren 1 und 2 zeigen eine flächige Plasmaerzeugungsvorrichtung in runder Form, wobei die Figur 1 die Plasmaerzeugungsvorrichtung in Explosionsdarstellung und die Figur 2 die Plasmaerzeugungsvorrichtung in zusammengefügter Darstellung zeigt.

Die Figuren 3 und 4 zeigen eine flächige Plasmaerzeugungsvorrichtung in eckiger Form, wobei die Figur 3 die Plasmaerzeugungsvorrichtung in Explosionsdarstellung und die Figur 4 die Plasmaerzeugungsvorrichtung in zusammengefügter Darstellung zeigt.

Die Figuren 5 und 6 zeigen ein Schema einer linearen Plasmaerzeugungsvorrichtung, wobei die Figur 5 die Plasmaerzeugungsvorrichtung mit schlauchförmiger Außenelektrode und die Figur 6 die Plasmaerzeugungsvorrichtung mit spiralförmiger Außenelektrode zeigt.

Die Figur 7 zeigt ein Plasmaerzeugungssystem mit mehreren Plasmaerzeugungsvorrichtungen mit rechteckiger Ausführung.

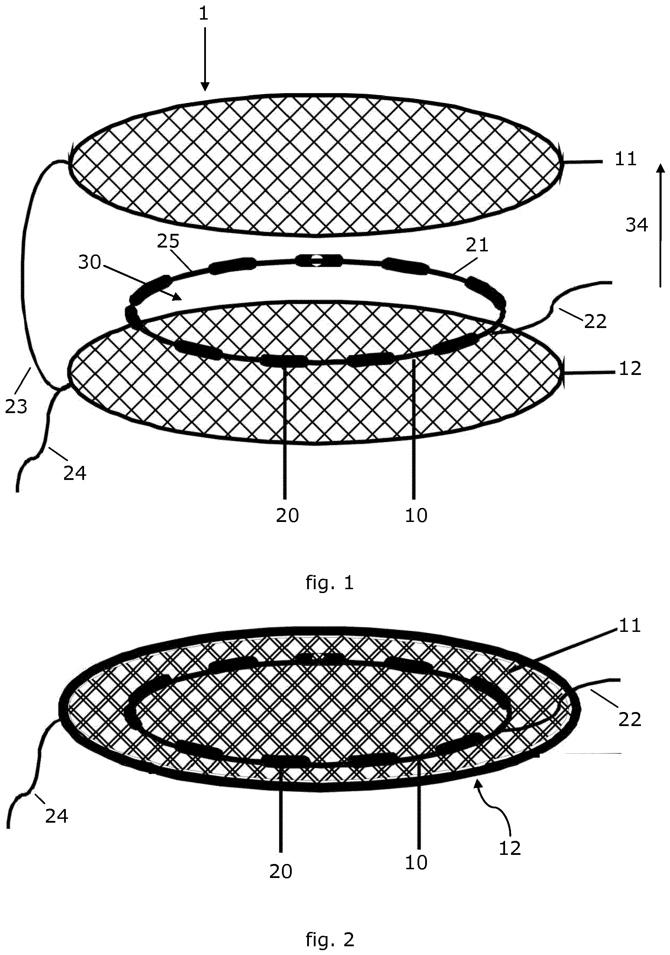

Die Figur 1 zeigt ein erstes Ausführungsbeispiel einer Plasmaerzeugungsvorrichtung 1 in Explosionsdarstellung. Die Plasmaerzeugungsvorrichtung 1 umfasst eine Fläche 30 umschließende Hochspannungselektrode 10. In beiden Richtungen der Normalen dieser eingeschlossenen Fläche 30 befindet sich jeweils eine Außenelektrode 11, 12. Die beiden Außenelektroden 11, 12 sind über eine elektrische Verbindung 23 miteinander verbunden. Die Hochspannungselektrode 10 ist in diesem Beispiel als Draht ausgeführt und wird durch die Anschlussleitung 22 gespeist. Die beiden Außenelektroden 11, 12 sind in diesem Beispiel als Metallgewebe ausgeführt und über das Massekabel 24 geerdet. Für eine dielektrisch behinderte Volumenentladung ist die Hochspannungselektrode 10 mit einem Dielektrikum 21 ummantelt. Um den für die dielektrisch behinderte Volumenentladung notwendigen äquidistanten Abstand 32 zwischen der, mit einem Dielektrikum 21 bedeckten, Hochspannungselektrode 10 und der geerdeten Außenelektrode 11 sicher zu stellen, ist die Hochspannungselektrode 10 mit Abstandselementen 20, sogenannten Spacern, bestückt. Die Abstandselemente 20 sind hier passende Silikonschlauchstücke, die jedoch nur an Abschnitten der Hochspannungselektrode 10 über deren Längserstreckung vorhanden sind, so dass dazwischen freie Abschnitte 25 verbleiben, in denen das Plasma erzeugt wird..

Im anwendbaren Zustand ist die Plasmaerzeugungsvorrichtung 1 entsprechend der Darstellung in Figur 2 ausgestaltet. Hierbei definieren die Abstandselemente 20 den Abstand zwischen den geerdeten Außenelektroden 11, 12 und der Hochspannungselektrode 10. Die in 2 dargestellte Plasmaerzeugungsvorrichtung 1 hat zwei Seiten, die durch die beiden Außenelektroden 11, 12 ausgebildet sind. Beide Seiten sind zum Auflegen auf eine zu behandelnde Wunde eines menschlichen Gewebes anwendbar, wobei zur besseren Verträglichkeit die Plasmaerzeugungsvorrichtung 1 mit einer hier nicht dargestellten dünnen, gasdurchlässigen Wundauflage kombiniert werden kann.

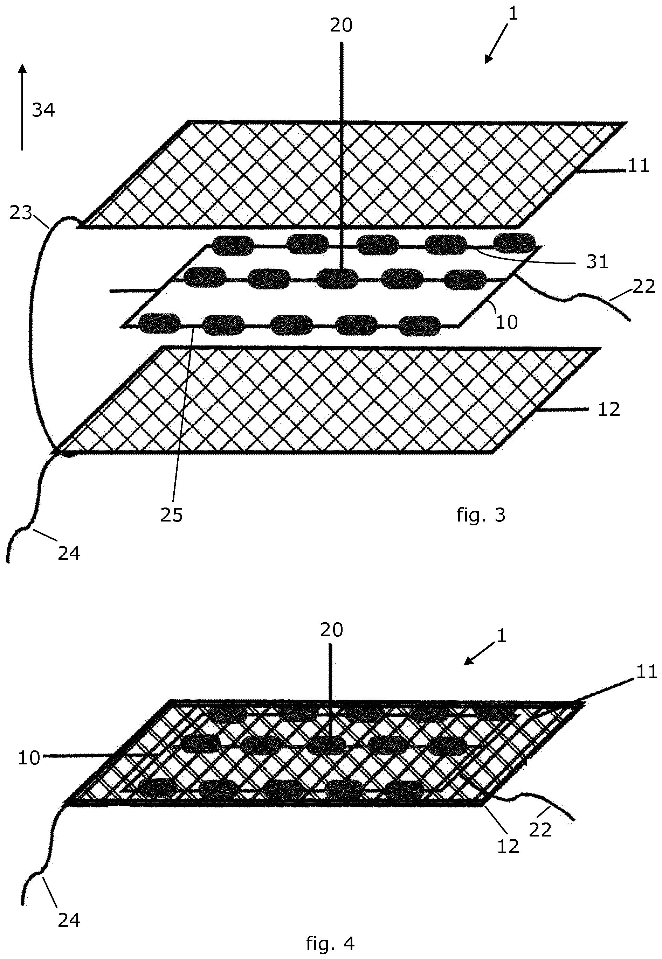

Eine weitere Ausführungsform in der Explosionsdarstellung zeigt Figur 3. Wie in dem in den Figuren 1 und 2 dargestelltem Ausführungsbeispiel gezeigt, ist hier die flächige Hochspannungselektrode 10 durch jeweils eine flächige Außenelektrode 11, 12 in beiden Richtungen der Normalen der durch die Hochspannungselektrode 10 eingeschlossenen Fläche 30 begrenzt. Des Weiteren sind auch in diesem Ausführungsbeispiel die Außenelektroden 11, 12 durch ein Metallgewebe ausgeführt. Beide Außenelektroden 11, 12 sind durch die elektrische Verbindung 23 miteinander verbunden und über das Massekabel 24 geerdet.

Im Gegensatz zu dem in den Figuren 1 und 2 dargestellten Ausführungsbeispiel ist hier die Plasmaerzeugungsvorrichtung 1 in einer rechteckigen Form ausgeführt. Das heißt, das Metallgewebe der beiden Außenelektroden 11, 12 hat eine rechteckige Ausführung. Die Hochspannungselektrode 10 mit der Anschlussleitung 22 hat im Gegensatz zu dem in den Figuren 1 und 2 dargestellten Ausführungsbeispiel parallele Abschnitte 31, so dass auch die Hochspannungselektrode 10 eine rechteckige Form ausbildet. Der für die dielektrisch behinderte Volumenentladung notwendige Abstand 32 wird auch hier durch Abstandselemente 20 gewährleistet. Die Abstandselemente 20 sind passende Silikonschlauchstücke .

Die Figur 4 zeigt das Ausführungsbeispiel der Plasmaerzeugungsvorrichtung 1 in der anwendbaren Form.

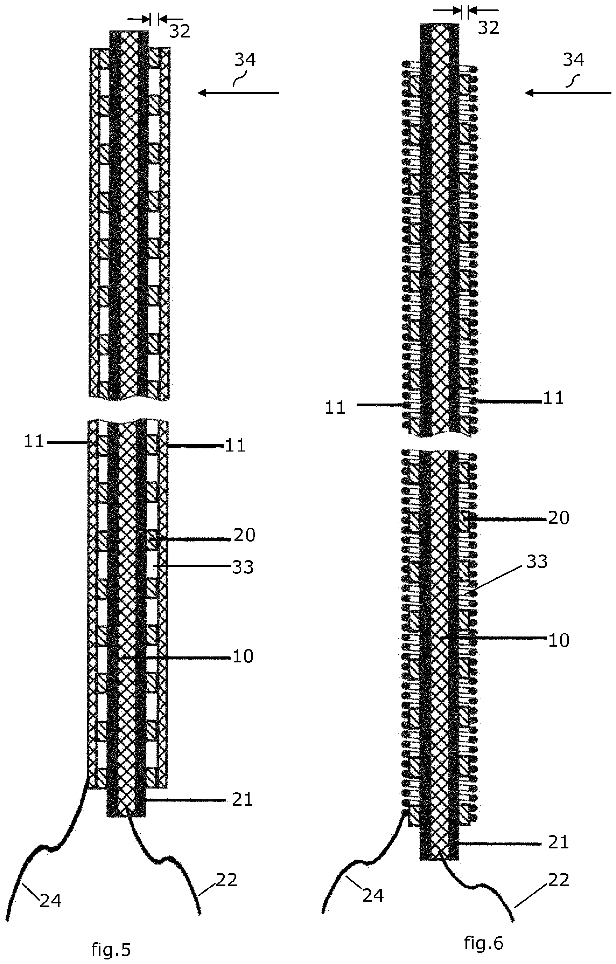

Ein Beispiel für eine lineare Ausführung einer erfindungsgemäßen Plasmaerzeugungsvorrichtung zeigt die Schnittdarstellung in Figur 5. In Figur 5 ist die drahtförmige Hochspannungselektrode 10 mit einem Dielektrikum 21 ummantelt. Die schlauchförmige Außenelektrode 11 umgibt die Hochspannungselektrode 10 und ist über das Massekabel 24 geerdet. Der für die dielektrisch behinderte Volumenentladung notwendige äquidistante Abstand 32 ist durch die Abstandselemente 20 gegeben. Das für die dielektrisch behinderte Volumenentladung erzeugte Atmosphärendruckplasma 33 befindet sich in dem Volumen zwischen den Abstandselementen 20, der geerdeten Außenelektrode 11 und dem Dielektrikum 21. Aus der dargestellten Koordinatenrichtung 34 ist ersichtlich, dass die Hochspannungselektrode 10 zumindest in der Koordinatenrichtung 34 zwischen Leitungsmaterial der Außenelektrode 11 angeordnet ist.

Eine weitere Ausführung der linearen Plasmaerzeugungsvorrichtung 1 ist in Figur 6 dargestellt. Die lineare Hochspannungselektrode 10, welche mit einem Dielektrikum 21 ummantelt ist, ist von einer spiralförmigen Außenelektrode 11 umgeben. Diese spiralförmige Außenelektrode 11 ist über das Massekabel 24 geerdet. Auch hier wird der äquidistante Abstand 32 zwischen der Hochspannungselektrode 10 und der Außenelektrode 11, der für die dielektrisch behinderte Volumenentladung notwendig ist, durch Abstandselemente 20 aufrechterhalten. Das Atmosphärendruckplasma 33 wird in dem durch die Außenelektrode 11, die Hochspannungselektrode 10 und den einzelnen Abstandselementen 20 begrenzten Räumen erzeugt.

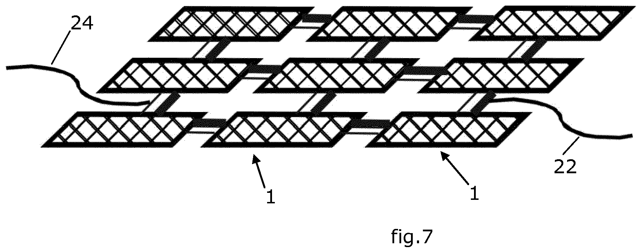

Die vorgenannten Ausführungsbeispiele beziehen sich auf die Ausführung einer einzelnen Plasmaerzeugungsvorrichtung 1. In Figur 7 ist ein Plasmaerzeugungssystem ersichtlich, welches mehrere Plasmaerzeugungsvorrichtungen, insbesondere zur großflächigen Behandlung von Oberflächen, aufweist.

In diesem Plasmaerzeugungssystem sind mehrere Plasmaerzeugungsvorrichtungen 1 in eckiger Form miteinander kaskadenförmig verbunden. Die zwischen den einzelnen Plasmaerzeugungsvorrichtungen 1 gezeigten Verbindungen führen zum einen die Hochspannung, welche mit der Anschlussleitung 22 verbunden ist, und zum anderen eine geerdete Verbindung, die mit dem Massekabel 24 verbunden ist.

**Bezugszeichenliste**

| | |
|---|---|
| Plasmaerzeugungsvorrichtung | 1 |
| Hochspannungselektrode | 10 |
| Erste Außenelektrode | 11 |
| Zweite Außenelektrode | 12 |
| Abstandselement | 20 |
| Dielektrikum | 21 |
| Anschlussleitung | 22 |
| Elektrische Verbindung | 23 |
| Massekabel | 24 |
| freier Abschnitt | 25 |
| Eingeschlossene Fläche | 30 |
| Parallelabschnitt | 31 |
| Abstand | 32 |
| Atmosphärendruckplasma | 33 |
| Koordinatenrichtung | 34 |

## Patentansprüche

1. Plasmaerzeugungsvorrichtung (1) umfassend eine Hochspannungselektrode (10) sowie mindestens eine Außenelektrode (11, 12), wobei die Hochspannungselektrode (10) zumindest in einer Koordinatenrichtung (34) zwischen Leitungsmaterial wenigstens einer Außenelektrode (11, 12) angeordnet ist und die Hochspannungselektrode (10) zumindest auf einer einer Außenelektrode (11, 12) der Plasmaerzeugungsvorrichtung (1) zugewandten Seite mit einem Dielektrikum (21) bedeckt ist, und wobei zwischen der jeweiligen Außenelektrode (11, 12) und der Hochspannungselektrode (10) über deren Längserstreckung wenigstens ein Abstandselement (20) vorhanden ist, welches zumindest im Bereich dessen Anordnung die jeweilige Außenelektrode (11,12) von der Hochspannungselektrode (10) elektrisch isoliert und welches die jeweilige Außenelektrode (11, 12) in einem konstanten Abstand von der Hochspannungselektrode (10) positioniert, **dadurch gekennzeichnet, dass** das Abstandselement (20) flächig ausgebildet ist, und dass das Abstandselement (20) eine perforierte und/ oder strukturierte, gasdurchlässige Folie aus einem elektrisch nicht leitfähigem Elastomer ist.

2. Plasmaerzeugungsvorrichtung nach Anspruch 1, **dadurch gekennzeichnet, dass** die Plasmaerzeugungsvorrichtung (1) eine erste Außenelektrode (11) und eine zweite Außenelektrode (12) umfasst, wobei die Hochspannungselektrode (10) zwischen den beiden Außenelektroden (11,12) angeordnet ist.

3. Plasmaerzeugungsvorrichtung nach einem der vorhergehenden Ansprüche, **dadurch gekennzeichnet, dass** die Hochspannungselektrode (10) als ein Draht ausgeführt ist.

4. Plasmaerzeugungsvorrichtung nach einem der vorhergehenden Ansprüche, **dadurch gekennzeichnet, dass** die Hochspannungselektrode (10) einen mäanderförmigen oder spiralförmigen Verlauf aufweist.

5. Plasmaerzeugungsvorrichtung nach einem der Ansprüche 1 bis 3, **dadurch gekennzeichnet, dass** die Hochspannungselektrode (10) als Geflecht, Gewebe, Gewirk oder Gestrick vorliegt.

6. Plasmaerzeugungsvorrichtung nach einem der vorhergehenden Ansprüche, **dadurch gekennzeichnet, dass** die Außenelektrode (11, 12) als Geflecht, Gewebe, Gewirk oder Gestrick vorliegt.

7. Plasmaerzeugungsvorrichtung nach einem der Ansprüche 1 bis 5, **dadurch gekennzeichnet, dass** die Außenelektrode (11 und 12) als elektrisch leitfähige Folie, insbesondere als perforierte und/oder strukturierte Folie, ausgebildet ist.

8. Plasmaerzeugungsvorrichtung nach einem der vorhergehenden Ansprüche, **dadurch gekennzeichnet, dass** die Plasmaerzeugungsvorrichtung (1) mehrere Abstandselemente (20) aufweist, die als die Hochspannungselektrode (10) zumindest abschnittsweise umschließende oder bedeckende Isolierstücke ausgeführt sind.

9. Plasmaerzeugungsvorrichtung nach einem der vorhergehenden Ansprüche , **dadurch gekennzeichnet, dass** die Folie Öffnungen aufweist.

10. Plasmaerzeugungsvorrichtung nach einem der Ansprüche 1 bis 9, **dadurch gekennzeichnet, dass** das Abstandselement (20) als ein Geflecht, Gewebe, Gewirk oder Gestrick ausgebildet ist.

11. Plasmaerzeugungsvorrichtung nach einem der vorhergehenden Ansprüche, **dadurch gekennzeichnet, dass** die Plasmaerzeugungsvorrichtung (1) eine Spannungsversorgungseinheit aufweist, die dazu eingerichtet ist, zwischen der Außenelektrode und der Hochspannungselektrode eine nieder- bis hochfrequente Hochspannung anzulegen.

12. Plasmaerzeugungssystem, insbesondere zur großflächigen Behandlung von Oberflächen, umfassend mehrere Plasmaerzeugungsvorrichtungen (1) gemäß einem der Ansprüche 1 bis 11 sowie eine oder mehrere Spannungsversorgungseinheiten, die mit den Plasmaerzeugungsvorrichtungen (1) elektrisch verbindbar oder verbunden sind.

13. Verfahren zur Erzeugung von Plasma, bei der eine Plasmaerzeugungsvorrichtung gemäß einem der Ansprüche 1 bis 11 oder auch ein Plasmaerzeugungssystem gemäß Anspruch 12 zur Verfügung gestellt wird und eine elektrische Hochspannung an die Hochspannungselektrode und mindestens eine Außenelektrode angelegt wird.

## Claims

1. A plasma generating device (1)
comprising a high-voltage electrode (10) as well as at least one external electrode (11, 12), wherein the high-voltage electrode (10) is, at least in one coordinate direction (34), arranged between conductive material of at least one external electrode (11, 12) and the high-voltage electrode (10) is covered with a dielectric (21) at least on one side facing an external electrode (11, 12) of the plasma generating device (1), and wherein between the respective external electrode (11, 12) and the high-voltage electrode (10) over its longitudinal extension at least one spacer element (20) is present, which at least in the region of its arrangement electrically insulates the respective external electrode (11, 12) from the high-voltage electrode (10) and which positions the respective external electrode (11, 12) at a constant distance from the high-voltage electrode (10), **characterized in that** the spacer element (20) is planar in design, and the spacer element (20) is a perforated and/or structured, gas-permeable foil made of an electrically non-conductive elastomer.

2. The plasma generating device according to Claim 1, **characterized in that** the plasma generating device (1) comprises a first external electrode (11) and a second external electrode (12), wherein the high-voltage electrode (10) is arranged between the two external electrodes (11, 12).

3. The plasma generating device according to any one of the preceding claims, **characterized in that** the high-voltage electrode (10) is configured as a wire.

4. The plasma generating device according to any one of the preceding claims, **characterized in that** the high-voltage electrode (10) has a meandering or spiraling course.

5. The plasma generating device according to any one of Claims 1 to 3, **characterized in that** the high-voltage electrode (10) is present as a mesh, woven fabric, knitted fabric or crocheted fabric.

6. The plasma generating device according to any one of the preceding claims, **characterized in that** the external electrode (11, 12) is present as a mesh, woven fabric, knitted fabric or crocheted fabric.

7. The plasma generating device according to any one of Claims 1 to 5, **characterized in that** the external electrode (11 and 12) is designed as an electrically conductive foil, in particular as a perforated and/or structured foil.

8. The plasma generating device according to any one of the preceding claims, **characterized in that** the plasma generating device (1) has several spacer elements (20) which are configured as insulation pieces enclosing or covering the high-voltage electrode (10) at least in sections.

9. The plasma generating device according to any one of the preceding claims, **characterized in that** the foil has openings.

10. The plasma generating device according to any one of Claims 1 to 9, **characterized in that** the spacer element (20) is designed as a mesh, woven fabric, knitted fabric or crocheted fabric.

11. The plasma generating device according to any one of the preceding claims, **characterized in that** the plasma generating device (1) has a voltage supply unit which is set up to apply a low to high-frequency high voltage between the external electrode and the high-voltage electrode.

12. A plasma generating system, in particular for large-scale treatment of surfaces, comprising several plasma generating devices (1) according to any one of Claims 1 to 11 as well as one or more voltage supply units which are electrically connected or connectible to the plasma generating devices (1).

13. A method for generating plasma, in which a plasma generating device according to any one of Claims 1 to 11 or a plasma generating system according to Claim 12 is provided and an electrical high voltage is applied to the high-voltage electrode and at least one external electrode.

## Revendications

1. Dispositif générateur de plasma (1)
comprenant une électrode à haute tension (10) ainsi qu'au moins une électrode extérieure (11, 12), dans lequel l'électrode à haute tension (10) est disposée au moins dans un axe de coordonnées (34) entre le matériau conducteur d'au moins une électrode extérieure (11, 12) et l'électrode à haute tension (10) est couverte d'un diélectrique (21) au moins sur une face tournée vers une électrode extérieure (11, 12) du dispositif générateur de plasma (1), et dans lequel au moins un élément d'écartement (20) est présent entre l'électrode extérieure respective (11, 12) et l'électrode à haute tension (10) sur leur extension longitudinale, lequel isole électriquement l'électrode extérieure respective (11,12) de l'électrode à haute tension (10) au moins dans la zone de son agencement et lequel positionne l'électrode extérieure respective (11, 12) à une distance constante de l'électrode à haute tension (10), **caractérisé en ce que** l'élément d'écartement (20) est conçu de manière plane et que l'élément d'écartement (20) est un film perforé et/ou structuré perméable aux gaz à base d'un élastomère électriquement non conducteur.

2. Dispositif générateur de plasma selon la revendication 1, **caractérisé en ce que** le dispositif générateur de plasma (1) comprend une première électrode extérieure (11) et une seconde électrode extérieure (12), dans lequel l'électrode à haute tension (10) est disposée entre les deux électrodes extérieures (11,12).

3. Dispositif générateur de plasma selon l'une quelconques des revendications précédentes, **caractérisé en ce que** l'électrode à haute tension (10) est réalisée sous la forme d'un fil métallique.

4. Dispositif générateur de plasma selon l'une quelconque des revendications précédentes, **caractérisé en ce que** l'électrode à haute tension (10) présente un parcours en forme de spirale ou de méandre.

5. Dispositif générateur de plasma selon l'une quelconque des revendications 1 à 3, **caractérisé en ce que** l'électrode à haute tension (10) existe sous la forme d'un treillis, d'une toile, d'un tissu à mailles ou d'un tricot.

6. Dispositif générateur de plasma selon l'une quelconque des revendications précédentes, **caractérisé en ce que** l'électrode extérieure (11, 12) existe sous la forme d'un treillis, d'une toile, d'un tissu à mailles ou d'un tricot.

7. Dispositif générateur de plasma selon l'une quelconque des revendications 1 à 5, **caractérisé en ce que** l'électrode extérieure (11 et 12) est constituée d'un film électriquement conducteur, notamment d'un film perforé et/ou structuré.

8. Dispositif générateur de plasma selon l'une quelconque des revendications précédentes, **caractérisé en ce que** le dispositif générateur de plasma (1) présente plusieurs éléments d'écartement (20), qui sont réalisés sous la forme de pièces isolantes entourant ou recouvrant l'électrode à haute tension (10) au moins par endroits.

9. Dispositif générateur de plasma selon l'une quelconque des revendications précédentes, **caractérisé en ce que** le film présente des ouvertures.

10. Dispositif générateur de plasma selon l'une quelconque des revendications 1 à 9, **caractérisé en ce que** l'élément d'écartement (20) est constitué d'un treillis, d'une toile, d'un tissu à mailles ou d'un tricot.

11. Dispositif générateur de plasma selon l'une quelconque des revendications précédentes, **caractérisé en ce que** le dispositif générateur de plasma (1) présente une unité d'alimentation électrique, qui est ajustée afin d'appliquer une haute tension de basse à haute fréquence entre l'électrode extérieure et l'électrode à haute tension.

12. Système générateur de plasma destiné notamment à traiter des surfaces très étendues, comprenant plusieurs dispositifs générateurs de plasma (1) selon l'une quelconques des revendications 1 à 11 ainsi qu'une ou plusieurs unités d'alimentation électrique, qui peuvent être ou sont reliées électriquement aux dispositifs générateurs de plasma (1).

13. Procédé servant à générer du plasma, au cours duquel un dispositif générateur de plasma selon l'une quelconques des revendications 1 à 11 ou aussi un système générateur de plasma selon la revendication 12 est mis à disposition et une haute tension électrique est appliquée sur l'électrode à haute tension et sur au moins une électrode extérieure.
